# EUROPÄISCHE PATENTANMELDUNG

(11) **EP 1 304 331 A1**
(43) Veröffentlichungstag der Anmeldung: **23.04.2003**
(21) Anmeldenummer: 02017352.2
(22) Anmeldetag: 02.08.2002
(51) Int. Cl.: C07D 495/02

(54) **Iminobiotinderivat**

(30) Priorität: 19.10.2001 DE 10151158
(71) Anmelder: Esplora GmbH, c/oTU Darmstadt Institut für Biochemie, 64287 Darmstadt (DE)
(72) Erfinder: Wolf, Sabine Dr.-Ing, 64853 Otzberg (DE); Bangsow, Thorsten Dr.-Ing., 64572 Büttelborn (DE); Jäger, Martina Dr.-Ing., 64367 Mühltal (DE); Oppolzer, Thomas Dipl.-Biol., 63222 Rödermark (DE)
(74) Vertreter: Weber, Dieter, Dr.

(57) **Zusammenfassung**

Die Erfindung betrifft Iminobiotinderivate, deren Herstellung sowie deren Verwendung zur Markierung von Proteinen und anderen Makromolekülen mit primärer Aminofunktion. Des weiteren betrifft die Erfindung ein Verfahren zur differentiellen qualitativen, semiquantitativen oder quantitativen Bestimmung von Proteinen oder anderen Makromolekülen mit primärer Aminofunktion in Zellen des gleichen Zelltyps, die sich in verschiedenen Zuständen befinden, oder in Zellen verschiedenen Zelltyps. Als Iminobiotinderivate, die in wäßrigem Lösungsmittel löslich sind, nicht membrangängig sind, ein Lösen der Bindung von einem festen Träger unter milden, schonenden, nicht denaturierenden Bedingungen erlauben, sterisch nicht gehindert und zur Markierung der extrazellulären Membrandomänen lebender Zellen geeignet sind, werden Verbindungen der allgemeinen Formel (I) vorgeschlagen, wobei x = 1-10 und y = 1-10 ist.

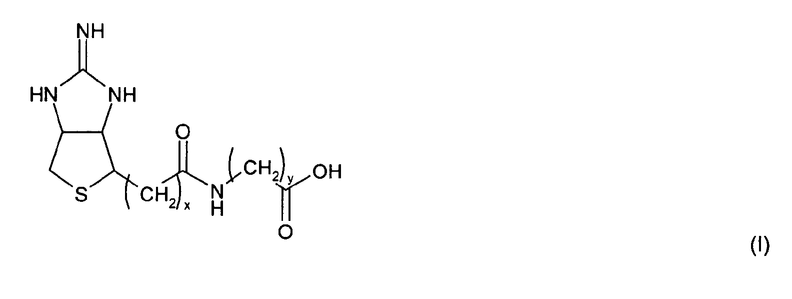

## Beschreibung

Die Erfindung betrifft Iminobiotinderivate, deren Herstellung sowie deren Verwendung zur Markierung von Proteinen und anderen Makromolekülen mit primärer Aminofunktion. Des weiteren betrifft die Erfindung ein Verfahren zur differentiellen qualitativen, semiquantitativen oder quantitativen Bestimmung von Proteinen oder anderen Makromolekülen mit primärer Aminofunktion in Zellen des gleichen Zelltyps, die sich in verschiedenen Zuständen befinden, oder in Zellen verschiedenen Zelltyps.

In vielen biochemischen Anwendungen wird die starke Bindung von Biotin (Vitamin H) an die Proteine Avidin bzw. Streptavidin ausgenutzt. Zielmoleküle, die Biotin oder Biotinderivate gebunden haben, können an festen Trägern, an denen wiederum Avidin oder Streptavidin fest gebunden ist, immobilisiert werden. Weitere Anwendungen sind z.B. die Präzipitation biotinylierter Zielmoleküle aus Lösungen mittels Avidin bzw. Streptavidin oder die affinitätschromatographische Reinigung biotinylierter Zielmoleküle über chromatographisches Säulenmaterial, an dem Avidin oder Streptavidin fest gebunden ist.

Handelsüblich sind verschiedene Biotinderivate erhältlich, die durch spezielle Aktivierungsgruppen die gezielte Kopplung des Biotinderivates an Biomoleküle ermöglichen. Ein Beispiel für eine solche Aktivierungsgruppe ist die aminreaktive Gruppe N-Hydroxysuccinimid (NHS), die Peptidbindungen vermitteln kann. Esterverbindungen aus Biotinderivaten und N-Hydroxysuccinimid sind jedoch nicht wasserlöslich und benötigen daher bei einer Reaktionsführung die Gegenwart von organischen Lösungsmitteln. Sind die Zielmoleküle jedoch empfindlich gegenüber organischen Lösungsmitteln oder sollen die Oberflächen lebender Zellen mit dem Biotinderivat gekoppelt werden, sind diese NHS-Biotinderivate nicht einsetzbar. Für solche Reaktionen können Biotinderivate mit der wasserlöslichen, aminreaktiven Gruppe Sulfo-N-Hydroxysuccinimid (Sulfo-NHS) eingesetzt werden. Für die Biotinylierung von Oberflächenproteinen lebender Zellen und anschließende Präzipitation ist beispielsweise das Reagens EZ-Link-Sulfo-NHS-LC-Biotin der Firma Pierce handelsüblich erhältlich (Abbildung 1). Aufgrund seiner Sulfo-NHS-Gruppe ist dieses Molekül nicht membrangängig und es besitzt einen Spacer-Arm oder Abstandhalter, der die Reaktion mit den Biomolekülen begünstigt, indem er sterische Hinderungen verringert.

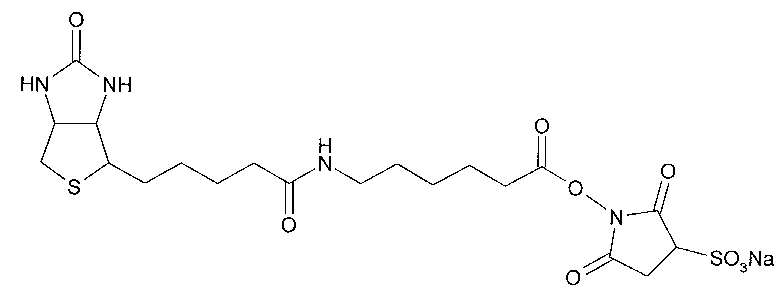

### Abbildung 1: EZ-Link-Sulfo-NHS-LC-Biotin

Ein Nachteil dieses bekannten Biotinderivates besteht in der äußerst hohen Bindungsaffinität des Biotins zu Avidin und Streptavidin. Die Interaktion dieser Moleküle kann nur unter extremen Bedingungen, z.B. mit 6-8 M Guanidinhydrochlorid bei pH 1,5, wieder gelöst werden. Für die affinitätschromatographische Reinigung biotinylierter Proteine ist dieses Reagens daher in vielen Fällen ungeeignet. Die extremen Bedingungen, die für die Elution des biotinylierten Proteins von der Chromatographiesäule benötigt werden, führen bei den meisten Proteinen zur Denaturierung und zur Schädigung des Säulenmaterials.

Als ein alternatives Biotinylierungsreagens steht Iminobiotin, das Guanidino-Analogon des Biotins, zur Verfügung Dieses weist ein vom pH-Wert abhängiges Bindeverhalten mit Avidin und Streptavidin auf (Abbildung 2).

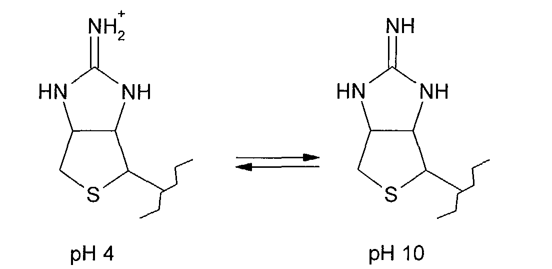

### Abbildung 2: pH-Abhängigkeit des Iminobiotins

Mit dem Iminobiotinderivat ist es möglich, Proteine affinitätschromatographisch unter wesentlich milderen Bedingungen zu reinigen. Da das Material der Affinitätssäule hierbei nicht geschädigt wird, sind mehrere Chromatographieläufe in unmittelbarer Folge auf einer Säule möglich. Neben der Schonung der eluierten Proteine bietet diese Methode somit auch die Vorteile der Zeit- und Kostenersparnis.

Als Reagens zur Iminobiotinylierung steht bisher nur EZ-Link-NHS-Iminobiotin der Firma Pierce zur Verfügung (Abbildung 3).

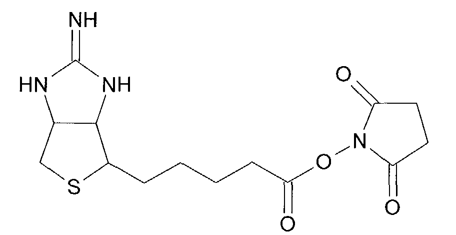

### Abbildung 3: EZ-Link-NHS-Iminobiotin

Dieser NHS-Iminobiotinester ist membrangängig. Dies hat bei der Markierung von Oberflächenproteinen lebender Zellen oder zumindest von Zellen mit intakter Membran den Nachteil, daß das Reagens in die Zellen eindringt und dann auch mit intrazellulären Proteinen umgesetzt wird.

Gygi et al., Nature Biotechnology, Band 17, Okt. 1999, S. 994-998, beschreiben ein Verfahren zur differentiellen quantitativen Bestimmung einzelner Proteine in einem Gemisch unter Verwendung eines Biotinderivates als Markierungsreagens für Proteine und unter Anwendung an sich bekannter massenspektrometrischer Verfahren. Das Markierungsreagens besitzt spezifische Reaktivität für Thiole und reagiert daher mit Cysteinresten der zu markierenden Proteine. Für die differentielle Analytik wird das Markierungsreagens in einer leichten und eine schwereren Form eingesetzt, wobei die schwerere Form achtfach deuteriert ist (D8), die leichte Form hingegen kein Deuterium enthält (D0). Das Markierungsreagens nach Gygi et al. ist in Abbildung 4 wiedergegeben.

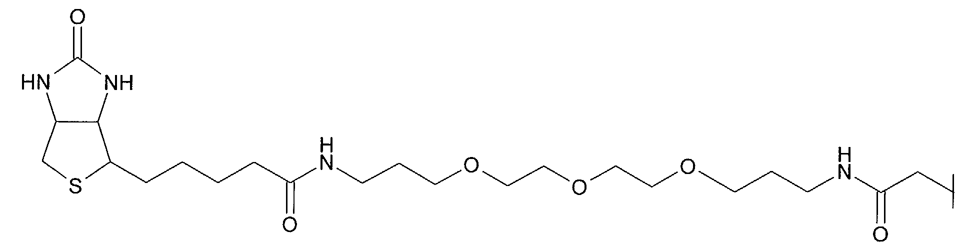

### Abbildung 4: Markierungsreagens nach Gygi et al.

Vor den Hintergrund des Standes der Technik bestand die Aufgabe der vorliegenden Erfindung daher darin, ein Reagens bereitzustellen, das in wäßrigem Lösungsmittel löslich ist, nicht membrangängig ist, ein Lösen der Bindung von einem festen Träger unter milden, schonenden, nicht denaturierenden Bedingungen erlaubt, sterisch nicht gehindert und zur Markierung der extrazellulären Membrandomänen lebender Zellen geeignet ist.

Diese Aufgabe wird erfindungsgemäß durch ein Iminobiotinderivat der allgemeinen Formel (I) gelöst, 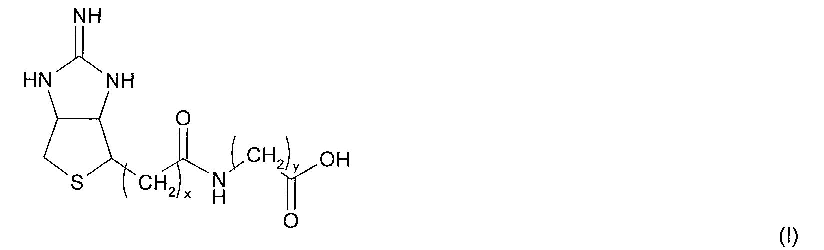 wobei x = 1 bis 10 und y = 1 bis 10 ist.

Des weiteren wird die erfindungsgemäße Aufgabe durch ein Iminobiotinderivat der allgemeinen Formel (II) gelöst, 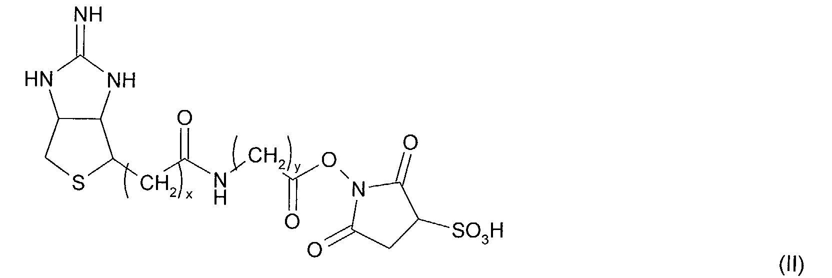 wobei x = 1 bis 10 und y = 1 bis 10 ist.

Die Reaktion der Markierung von Proteinen oder Makromolekülen mit primärer Aminofunktion mit dem Iminobiotinderivat der Formel (I) verläuft über das Iminobiotinderivat der Formel (II) als Zwischenprodukt. Beide Iminobiotinderivate sind daher als Markierungsreagenzien geeignet. Das Iminobiotinderivat der Formel (I) läßt sich als stabile, handelsfähige Verbindung herstellen. Das Iminobiotinderivat der Formel (II) stellt die aminreaktive, aktivierte Form des Iminobiotinderivates der Formel (I) dar. Es ist aufgrund seines aktivierten Zustandes weniger stabil und daher als Handelsform schlechter geeignet. Seine Halbwertszeit liegt im Bereich von ca. 30 Minuten, je nach Lagerungsbedingungen. Da es das eigentliche Markierungsreagens darstellt, welches mit einer primären Aminofunktion eines Proteins oder eines anderen Makromoleküls reagiert, ist es Gegenstand der Erfindung, auch wenn es aus einem anderen Edukt als dem Iminobiotinderivat der Formel (I) hergestellt wird.

Die erfindungsgemäßen Iminobiotinderivate der Formeln (I) und (II) sind wasserlöslich, so daß die Verwendung organischer Lösungsmittel vermieden wird. Die Iminobiotingruppe erlaubt es im Gegensatz zur Biotingruppe, die Bindung an Avidin oder Streptavidin unter milden Bedingungen durch Veränderung des pH-Wertes unter milden Bedingungen zu stabilisieren oder zu lösen. Das erfindungsgemäße Iminobiotinderivat ist nicht membrangängig, so daß es sich besonders vorteilhaft zur Markierung von Oberflächenproteinen lebender Zellen bzw. von Zellen mit intakter Zellmembran eignet. Durch entsprechende Auswahl der Kettenlängen der beiden Kohlenwasserstoffketten (CH₂)ₓ und (CH₂)_{y} läßt sich die Länge des Spacer-Arms zwischen der Iminobiotingruppe und dem gebundenen Protein optimal einstellen, um möglichst hohe Stabilität bei geringer sterischer Hinderung zu erzielen.

Bei einer bevorzugten Ausführungsform der erfindungsgemäßen Iminobiotinderivate der Formeln (I) und (II) ist x = 4 und/oder y = 5.

Ganz besonders bevorzugt ist es, wenn x = 4 und y = 5 ist. Die entsprechenden Iminobiotinderivate sind nachfolgend als Formeln (la) (Iminobiotin-Aminocapronsäure = ImBAS) und (IIa) (Sulfo-NHS-Iminobiotin-Aminocapronsäure = Sulfo-NHS-ImBAS) wiedergegeben (Abbildungen 5a und 5b).

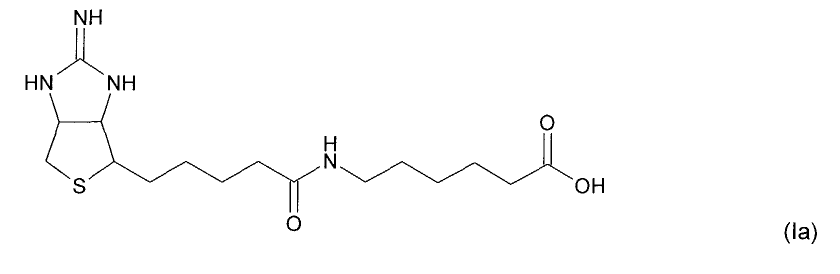

### Abbildung 5a: Iminobiotin-Aminocapronsäure (ImBAS)

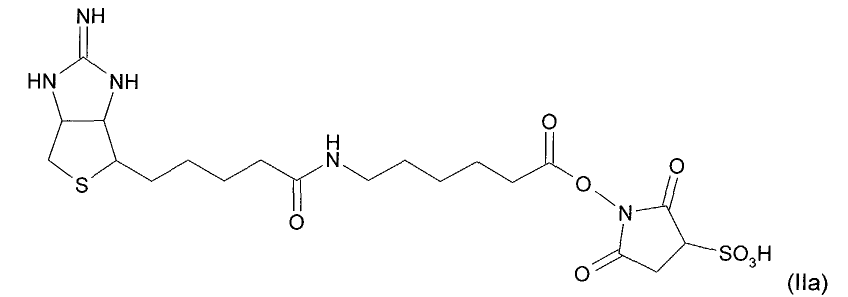

### Abbildung 5b: Sulfo-NHS-lminobiotin-Aminocapronsäure (Sulfo-NHS-ImBAS)

Bei einer weiteren bevorzugten Ausführungsform der Erfindung ist/ sind in dem Iminobiotinderivat eines oder mehrere der Wasserstoffatome (H) durch Deuterium (D) oder gegebenenfalls auch durch Tritium (T) ersetzt. Die deuterierte Form des erfindungsgemäßen Iminobiotinderivates eignet sich für die qualitative, semiquantitative und quantitative Proteinanalytik mittels massenspektrometrischer Verfahren. Zur Differenzierung wird das Iminobiotinderivat in der nicht deuterierten Form eingesetzt. Beispiele für differentielle Proteinanalytik sind in der oben genannten Veröffentlichung von Gygi et al., a.a.O. beschrieben und sollen durch Verweis zum Inhalt dieser Anmeldung gehören.

Die Erfindung umfaßt weiterhin ein Verfahren zur Herstellung erfindungsgemäßen Iminobiotinderivates nach Formel (I) bzw. (la) mit der Stufe, in der man ein Iminobiotinderivat der allgemeinen Formel (III) mit einer Aminocarbonsäure der allgemeinen Formel (IV) umsetzt, 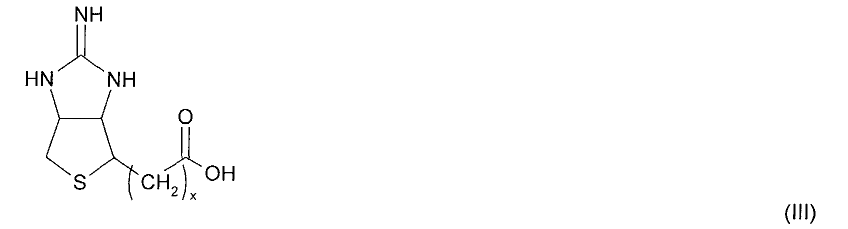 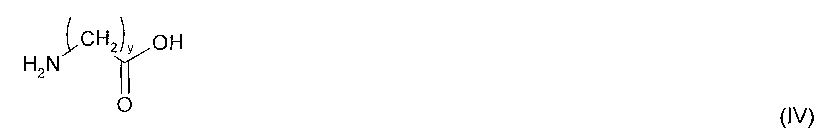 wobei x und y wie oben definiert sind.

Bei der besonders bevorzugten Ausführungsform mit x=4 und y=5 ist die Verbindung der Formel (IV) 6-Aminocapronsäure. Vorteilhaft ist es, die Umsetzung in phosphatgepufferter, wäßriger Lösung bei einem pH-Wert von 6,0 bis 8,5 für 30 Minuten bis 5 Stunden bei 0°C bis 50°C durchzuführen.

Erfindungsgemäß wird das Iminobiotinderivat der Formel (II) bzw. (lla) vorzugsweise als aktiviertes Zwischenprodukt bei der Markierungsreaktion in situ hergestellt, indem man das Iminobiotinderivat der Formel (I) bzw. (la) mit 1-Ethyl-3-(3-dimethylaminopropyl)-carbodiimid (EDC) und Sulfo-N-hydroxysuccinimid (Sulfo-NHS) umsetzt.

Die Markierung von Proteinen bzw. Makromolekülen mit primärer Aminofunktion schließt sich an die Herstellung des aktivierten Sulfo-NHS-lminobiotinderivates der Formel (II) bzw. (lla) an, indem man es mit Proteinen bzw. Makromolekülen mit primärer Aminofunktion in dem Ansatz umsetzt. Die Proteine können dem Ansatz in beliebiger Form zugegeben werden. Die Proteine können in Lösung oder in trägergebundener Form vorliegen. Zur spezifischen Markierung von Membranoberflächenproteinen lebender oder zumindest intakter Zellen wird die Markierung in Gegenwart der ganzen Zellen durchgeführt. Nach Abschluß der Markierungsreaktion werden die Zellen lysiert, membrangebundene Proteine gegebenenfalls durch einen zusätzlichen Verfahrensschritt aus der Membran gelöst und die markierten Membranproteine von den nicht markierten intrazellulären Proteinen und von übrigen Zellbestandteilen und -trümmern abgetrennt und gereinigt. Die Abtrennung und Reinigung der markierten Proteine erfolgt vorzugsweise affinitätschromatographisch unter Ausnutzung der Bindungsaktivität der Iminobiotingruppe an Avidin bzw. Streptavidin.

Die erfindungsgemäße Markierung von Proteinen ist nachfolgend ausgehend von der Iminobiotin-Aminocapronsäure gemäß Formel (la) über den Ester Sulfo-NHS-lminobiotinyl-Aminocapronsäure gemäß Formel (lla) in Abbildung 6 dargestellt. Zunächst reagiert EDC mit der Carboxylgruppe der Iminobiotinyl-Aminocapronsäure und bildet ein labiles Intermediat (nicht dargestellt). Dieses Intermediat wird durch Sulfo-NHS stabilisiert, und es bildet sich der Ester Sulfo-NHS-lminobiotinyl-Aminocapronsäure. Zur Markierung von Proteinen bzw. Zellen können diese dem Ansatz ohne eine vorherige Aufreinigung des Sulfo-NHS-Derivates zugegeben werden.

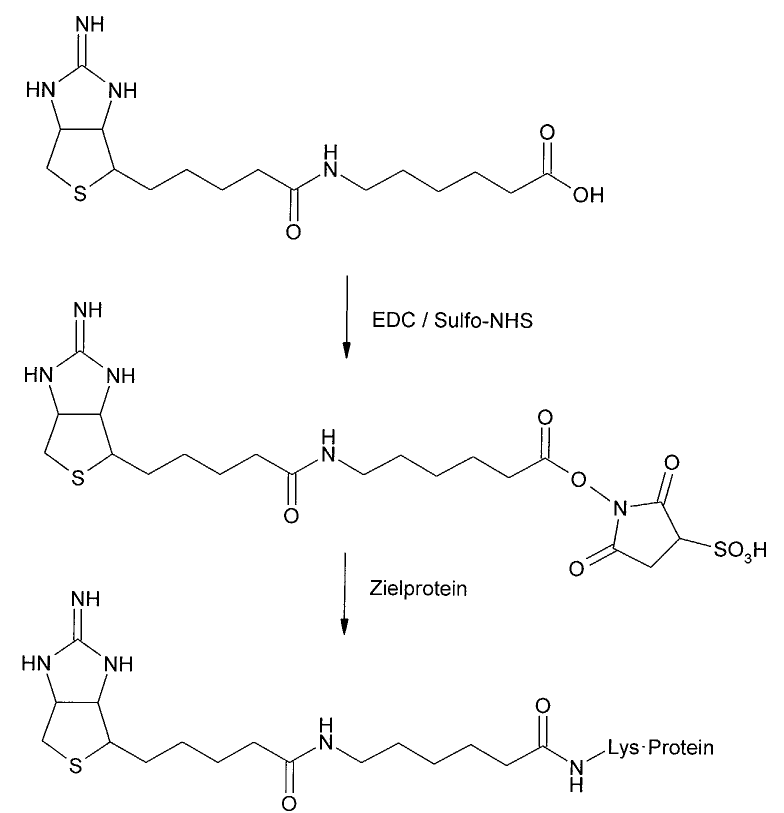

### Abbildung 6: Markierungsreaktion

Die Erfindung umfaßt auch ein Verfahren zur differentiellen qualitativen, semiquantitativen oder quantitativen Bestimmung von Proteinen oder anderen Makromolekülen mit primärer Aminofunktion in Zellen des gleichen Zelltyps, die sich in verschiedenen Zuständen befinden, oder in Zellen verschiedenen Zelltyps mit den Stufen, in denen man
a) Proteine oder Makromoleküle der Zellen mit dem Iminobiotinderivat der Formel (II) markiert, wobei man für Zellen in verschiedenen Zuständen bzw. für verschiedene Zelltypen jeweils verschiedene Isotopomere der Iminobiotinderivate mit verschiedenen Molekulargewichten einsetzt,
b) gegebenenfalls die markierten Proteine oder Makromoleküle aus Zellen verschiedener Zustände bzw. aus verschiedenen Zelltypen vereinigt und
c) die markierten Proteine oder Makromoleküle affinitätschromatographisch reinigt und/oder anreichert,
d) die markierten Proteine oder Makromoleküle mittels massenspektrometrischer Methoden analysiert.

Im Gegensatz zur Untersuchung des Proteinexpressionsmusters von Zellen auf mRNA-Ebene befaßt sich die Proteomanalyse mit der quantitativen Bestimmung der exprimierten Proteine als solche. Zur Analyse des Proteinexpressionsmusters werden zweidimensionale Gelelektrophorese (2D-PAGE) und Massenspektrometrie eingesetzt. Bei der 2D-PAGE wird zunächst eine isoelektrische Fokussierung durchgeführt, gefolgt von SDS-Gelelektroforese (SDS = Natriumdodecylsulfat). Hierdurch ist es möglich, hochkomplexe Proteingemische, wie z.B. Zellysate, zunächst nach den proteinspezifischen isoelektrischen Punkten und anschließend nach Proteingröße zu trennen. Eine sich anschließende proteolytische Spaltung ermöglicht die massenspektrometrische Identifizierung der getrennten Proteine. Allerdings weist die 2D-PAGE spezifische Limitationen auf. So sind gerade Membranproteine, die für Zell-Zell-Interaktionen und Transportprozesse von entscheidender Bedeutung sind, mit dieser Technik schwer aufzulösen. Zudem ist die Visualisierung von Proteinen, die nur in geringer Kopienzahl in Zellen vorliegen, sehr problematisch.

Mit Hilfe der erfindungsgemäßen Iminobiotinderivate ist es nun möglich, Proteine bzw. Makromoleküle mit primärer Aminofunktion, die sich auf der Außenseite der Zellmembran intakter Zellen präsentieren, zu markieren. Da die erfindungsgemäßen Iminobiotinderivate nicht membrangängig sind, bleiben die intrazellulären Moleküle unmarkiert. Nach der Markierung werden die Zellen lysiert, die markierten Moleküle in Lösung gebracht, affinitätschromatographisch angereichert und massenspektrometrisch analysiert. Bei Fragestellungen der Proteomanalyse werden in der Regel differentielle Analyseansätze eingesetzt, bei denen die Proteinexpressionsmuster unterschiedlicher Zustände eines Zelltyps oder unterschiedlicher Zelltypen miteinander verglichen werden. Bei dem erfindungsgemäßen differentiellen Analyseverfahren werden verschiedene Isotopomere des gleichen Iminobiotinderivats, vorzugsweise Iminobiotinaminocapronsäure (ImBAS), eingesetzt. Erfindungsgemäß besonders bevorzugt verwendet man Isotopomere des ImBAS, die sich lediglich in ihrem Deuteriumgehalt unterscheiden, so daß beide Verbindungen zwar chemisch fast identisch sind, sich aber in ihrer Masse meßbar unterscheiden. Für die Markierung beispielsweise zweier unterschiedlicher Zelltypen zur Charakterisierung von Unterschieden in der Proteinexpression werden die Proteine eines der beiden Zelltypen vorzugsweise mit dem nicht deuterierten Isotopomeren des Markierungsmittels und der andere Zelltyp mit der deuterierten Form markiert. Zur Unterscheidung werden hierin die Bezeichnungen D0-Isotopomer für die nicht deuterierte Form und DX-lsotopomer für die deuterierte Form verwendet, wobei X für die Anzahl an Deuteriumatomen in einem Molekül des Iminobiotinderivates steht. Um einen ausreichenden Massenunterschied zu erreichen, der gut meßbar ist, sollte das deuterierte Molekül erfindungsgemäß vorzugsweise wenigstens das D6-lsotopomere, besser noch wenigstens das D8-Isotopomere sein. Erfindungsgemäß besonders bevorzugt verwendet man das D10-Isotopomere des ImBAS. Im nachstehenden erfindungsgemäßen Beispiel werden die D0- und D10-Isotopomeren des ImBAS verwendet, die im Massenspektrum eine Masse/Ladungs-Differenz von 10 Thomson aufweisen (bei einfach geladenem Molekül; Thomson = Masse/Ladung). Bei mehrfach geladenen Molekülen erscheint die Masse/Ladungs-Differenz im Massenspektrum entsprechend geringer.

### Beispiel 1: Differentielle Analyse des Proteinexpressionsmusters

In diesem Beispiel werden in einem differentiellen Analyseansatz die Oberflächenproteine von Endothelzellen verschiedener Herkunft mit je einem Isotopomeren des ImBAS (D0 bzw. D10) markiert. Die Endothelzellen werden vereinigt, aufgeschlossen und die markierten Oberflächenmoleküle werden angereichert. Nach der Anreicherung wird der Ansatz massenspektrometrisch analysiert. Unterschiede der Proteinexpression der verschiedenen Zellen zeigen sich durch Unterschiede der Peaks für ein einzelnes Protein. Aufgrund des Massenunterschiedes der Markierungsreagenzien, mit denen die Proteine markiert sind, lassen sich die einzelnen Proteinpeaks der jeweiligen Probe von Zellen verschiedener Herkunft zuordnen.

Die Analyse gliedert sich in die folgenden Unterpunkte:
a) Markierung der Zellen mit D0-ImBAS oder D10-ImBAS;
b) Aufschluß der markierten Zellen;
c) Bromcyanspaltung der unlöslichen Bestandteile des Aufschlusses;
d) Affinitätsreinigung und Anreicherung der markierten Biomoleküle; und
e) Analyse der markierten Biomoleküle mittels massenspektrometrischer Methoden.

### a) Markierung der Zellen mit D0-ImBAS oder D10-ImBAS

Die Markierung der zellulären Membranproteine mit ImBAS beruht auf der oben beschriebenen zweistufigen Reaktion in Gegenwart von EDC und Sulfo-NHS. Ein Reaktionsansatz mit einem Gesamtvolumen von 400 µl mit 21 mM ImBAS, 25 mM Sulfo-NHS und 10 mM EDC in phosphatgepufferter physiologischer Kochsalzlösung (PBS; pH 7,2) wird 30 Minuten bei Raumtemperatur geschüttelt. Die Umsetzung von ImBAS zu Sulfo-NHS-ImBAS kann optional durch Einstellen von 0,5 mM β-Mercaptoethanol gestoppt werden. Die zulässige Konzentration an β-Mercaptoethanol ist dabei zelltypspezifisch. Der Ansatz wird nach Zugabe des β-Mercaptoethanols weitere 5 Minuten geschüttelt.

Im zweiten Reaktionsschritt werden dem Ansatz die zu markierenden Zellen in einer Menge von etwa 5 x 10⁷ zugegeben, der Ansatz mit PBS-Puffer auf 2 ml aufgefüllt und für eine Stunde bei 4°C auf einem Kippschüttler inkubiert. Die Zellen werden anschließend bei 100 x g sedimentiert und mit Waschpuffer (40 mM Glyzin in PBS-Puffer) gewaschen, um die Reaktion zu stoppen. Anschließend werden die Zellen zweimal mit PBS-Puffer gewaschen.

### b) Aufschluß der markierten Zellen

Ziel des Aufschlusses ist es, die markierten Proteine in Lösung zu bringen, um sie chromatographischen Methoden zugänglich zu machen. Im Rahmen der differentiellen Analyse können die unterschiedlich markierten Zellen bereits vor dem Aufschluß vereinigt werden, um sicherzustellen, daß die Proben identische Aufschlußbedingungen erfahren.

Der Aufschluß der markierten Zellen erfolgt in PBS-Puffer mit 2% (v/v) Triton X-100 und einem Proteaseinhibitor-Mix (Complete-Mini, Firma Roche). Das Zellsediment eines Ansatzes wird in 1 ml dieses Aufschlußpuffers resuspendiert und wie folgt behandelt: 60 Sekunden Vortexen, dreimal 10 Sekunden mit Ultraschall behandeln (zwischendurch auf Eis kühlen), 60 Minuten auf Eis inkubieren, dreimal 10 Sekunden mit Ultraschall behandeln (zwischendurch auf Eis kühlen), 60 Sekunden Vortexen, unlösliche Bestandteile bei 40.000 x g sedimentieren.

Der Überstand der Zentrifugation enthält die unter diesen Bedingungen löslichen Biomoleküle, die nun der Affinitätschromatographie zugänglich sind.

### c) Bromcyanspaltung der unlöslichen Bestandteile des Aufschlusses

Das Sediment des Aufschlusses enthält neben DNS und Membranfragmenten auch unlösliche markierte Membranproteine. Ziel der Bromcyanspaltung ist es, diese Proteine bevorzugt an ihren hydrophoben Sequenzabschnitten zu hydrolysieren, um lösliche Peptide dieser Proteine zu erhalten. Die Bromcyanspaltung spaltet selektiv C-Terminal der relativ seltenen Aminosäure Methionin. Vor der Hydrolyse wird das Sediment mit Benzonase behandelt, um DNS in dem Sediment zu spalten. Des weiteren erfolgt vor der Bromcyanspaltung eine Reduktion der oxidierten Methioninreste.

Das Sediment eines Aufschlusses wird in 200 µl Benzonasepuffer (50 mM Tris-HCI, pH 8, 1 mM MgCl₂) aufgenommen. Anschließend werden 2 µl Benzonase (Firma Merck) zugegeben und der Ansatz für zwei Stunden bei 37°C inkubiert. Danach wird der Ansatz auf 5% (v/v) Natriumdodecylsulfat (SDS) eingestellt und 20 Minuten bei 96°C inkubiert. Abschließend werden die Proteine mit Aceton gefällt.

Die gefällten Proteine werden in 1,5 ml Reduktionslösung (2,9 M β-Mercaptoethanol, 5% (v/v) Essigsäure) aufgenommen, für eine Minute mit Argon (oder Stickstoff) begast und für 12 Stunden bei 37°C inkubiert. Anschließend wird die Probe bis zur Trockene eingeengt.

Die Probe wird nun in 1 ml 70% (v/v) Ameisensäure aufgenommen und 100 µl Bromcyanlösung (660 mM Bromcyan, 60,1% (v/v) Ameisensäure) zugegeben. Es folgt eine Inkubation bei Raumtemperatur im Dunkeln für 24 Stunden. Die Hydrolyse wird anschließend durch Einengen bis zur Trockene beendet.

Die so erhaltenen Peptide sind zum größten Teil in dem in der Affinitätschromatographie verwendeten Puffer löslich und daher dieser Trennmethode zugänglich.

### d) Affinitätsreinigung und Anreicherung der markierten Biomoleküle

Mittels Affinitätschromatographie an immobilisiertem Streptavidin ist es möglich, mit ImBAS markierte Proteine und Peptide unter milden Bedingungen zu reinigen und anzureichern.

Als chromatographisches Säulenmaterial wird eine HiTrap-Streptavidinsäule (Firma Amersham Pharmacia Biotech) eingesetzt. Die Proben werden in Bindepuffer (50 mM Ammoniumhydrogencarbonat, 500 mM NaCI, 0,05% (v/v) Triton X-100, pH 10) nach Möglichkeit vollständig gelöst. Die trokkenen Peptidproben werden hierzu direkt im Bindepuffer aufgenommen und anschließend zentrifugiert oder sterilfiltriert, um mögliche unlösliche Bestandteile abzutrennen. Der Überstand des Aufschlusses enthält gelöste Proteine, die zwecks Pufferwechsels über einer Membran eingeengt oder gefällt werden, um sie dann mit Bindepuffer zu lösen.

Zur Vorbereitung der Affinitätschromatographie wird das Säulenmaterial zunächst mit dem fünffachen Säulenvolumen an Bindepuffer equilibriert. Anschließend wird die Probe aufgetragen und die nicht bindenden Bestandteile der Proben mit dem zweifachen Säulenvolumen an Bindepuffer ausgewaschen. Mit dem fünffachen Säulenvolumen an Elutionspuffer (50 mM Ammoniumacetat, 500 mM NaCl, 0,05% (v/v) Triton X-100, pH 4) werden die Proteine von dem Säulenmaterial eluiert. Das Elutionsprofil wird dabei am Säulenauslauf durch Messung der Lichtabsorption bei 280 nm verfolgt und das Eluat gesammelt, welches die markierten Oberflächenproteine bzw. deren Peptide in reiner und angereicherter Form enthält.

Da das Material der Affinitätssäule bei dem Verfahren nicht geschädigt wird, sind mehrere Chromatographieläufe in unmittelbarer Folge auf derselben Säule möglich. Neben der schonenden Elution der Proteine bzw. Peptide spart dieses Verfahren auch Zeit und Kosten.

### e) Analyse der markierten Proteine und Peptide mittels massenspektrometrischer Methoden

Die bei der affinitätschromatographischen Reinigung erhaltenen Eluate enthalten die isolierten Proteine bzw. Peptide der unterschiedlichen Zellzustände, die jeweils mit einem der beiden Isotopomeren des ImBAS markiert sind. Die angereicherten Peptide der Bromcyanspaltung können direkt für die massenspektrometrische Untersuchung eingesetzt werden. Alternativ können sie aber auch einer weiteren proteolytischen Hydrolyse (z.B. mit Trypsin) unterzogen werden. Dieser Hydrolyse kann bei Bedarf eine Polyacrylamidgelelektrophorese vorgeschaltet werden, um die Komplexität der Proben zu verringern.

Für die massenspektrometrische Analyse der Proben ist sowohl die Matrix Assisted Laser Desorption lonization (MALDI) als auch die Elektrospray-lonisierung (ESI) geeignet. Die markierten Peptide der unterschiedlichen Zellzustände erscheinen im Massenspektrum jeweils mit einer Differenz von 10 Thomson, wenn sie eine einfache Ladung tragen (MALDI). Bei mehrfacher Ladung, z.B. zwei- bis dreifacher Ladung, ist die Differenz im Massenspektrum entsprechend geringer. Da beide ImBAS-Varianten chemisch äquivalent sind, kann aus dem Verhältnis zweier zum gleichen Peptid gehörender Signale (ImBAS D0-markiert zu ImBAS D10-markiert) eine quantitative Aussage über das Expressionsmuster der Proteine in den unterschiedlichen Zellzuständen gemacht werden.

Bei Proben hoher Komplexität kann auch ein ESI-Massenspektrometer mit vorgeschalteter Probenauftrennung über HPLC (High Performance Liquid Chromatography) eingesetzt werden. Für die Analyse von unbekannten Proteinen kann auch Tandemmassenspektrometrie (MS/MS) angewendet werden. Das erfindungsgemäße Verfahren ermöglicht es, die Biomoleküle der Membranaußenseite intakter Zellen selektiv zu markieren, in Lösung zu bringen, über Affinitätschromatographie anzureichern und mittels Massenspektrometrie zu analysieren.

### Beispiel 2: Herstellung von Iminobiotinyl-Aminocapronsäure

In einem Volumen von 50 ml werden 200 mg 6-Aminocapronsäure (30,5 mM, 131,1 g/mol) und 50 mg NHS-lminobiotin (2,3 mM, 436,4 g/mol als Trifluoroacetamidverbindung) in Phosphatpuffer (pH 7,2) für zwei Stunden bei 4°C umgesetzt. Bei der eingesetzten 6-Aminocapronsäure handelt es sich wahlweise um nicht deuterierte 6-Aminocapronsäure oder um das deuterierte Isotopomer. Die hierin eingesetzte D10-markierte 6-Aminocapronsäure ist in ihrer Lactam-Form erhältlich (Firma Cambridge Isotope Laboratories). Um das Iminobiotin (Firma Pierce) in Lösung zu bringen, wird es zunächst in 400 µl Dimethylformamid gelöst und dann dem Ansatz zugegeben.

Anschließend wird der Reaktionsansatz zur Trockene eingeengt, die Iminobiotinyl-Aminocapronsäure in 5 ml Ethanol gelöst und von der im Überschuß zugegebenen 6-Aminocapronsäure, die nicht in Ethanol löslich ist, abgetrennt. Die gelöste Iminobiotinyl-Aminocapronsäure wird erneut bis zur Trockene eingeengt. Der Waschschritt mit Ethanol wird wiederholt. Das Produkt wird mittels Umkehr-Phasen-Chromatographie an C2/C18-Material gereinigt, um mögliche Nebenprodukte (z.B. Iminobiotin) abzutrennen.

### NMR-Daten:

¹H-NMR ([D4] Methanol δ = 4,72 (dd, ³J = 7,9, ³J = 4,5, 1H, -CH-); 4,54(dd, ³J = 7,9, ³J = 4,5, 1H, -CH-); 3,25-3,3 (m, 1H, -SCH-); 3,18 (t, 2H, ³J = 6,8, -OCNCH₂-); 2,8-3,05 (m, 2H, -SCH₂CH-); 2,27 (t, 2H ³J = 7,3, - -OCCH₂CH₂-); 2,21 (t, ³J = 7,3, 2H, -OCCH₂CH₂-); 1,3-1,8 (m, -CH₂-, 12H)

### MALDI-Daten:

MS (MALDI): 357,19 (M+H⁺)

### Schmelzpunkt:

Zersetzung ab 156°C

## Patentansprüche

1. Iminobiotinderivat der allgemeinen Formel (I) 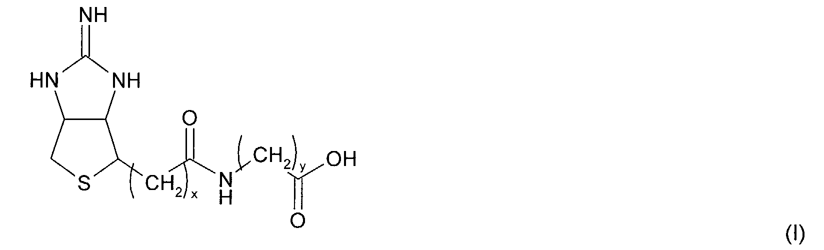 wobei x = 1 bis 10 und y = 1 bis 10 ist.

2. Iminobiotinderivat der allgemeinen Formel (II) 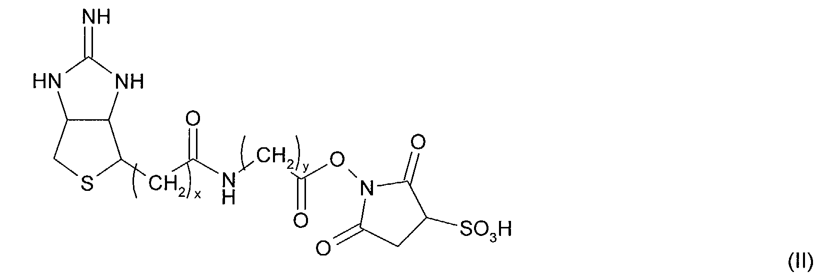 wobei x = 1 bis 10 und y = 1 bis 10 ist.

3. Iminobiotinderivat nach einem der Ansprüche 1 oder 2, **dadurch gekennzeichnet, daß** x = 4 und/ oder y = 5 ist.

4. Iminobiotinderivat nach einem der Ansprüche 1 bis 3, **dadurch gekennzeichnet, daß** eines oder mehrere der Wasserstoffatome (H) durch Deuterium (D) oder Tritium (T) ersetzt ist/sind.

5. Verfahren zur Herstellung eines Iminobiotinderivates nach einem der Ansprüche 1, 3 oder 4, mit der Stufe, in der man ein Iminobiotinderivat der allgemeinen Formel (III) mit einer Aminocarbonsäure der allgemeinen Formel (IV) umsetzt, 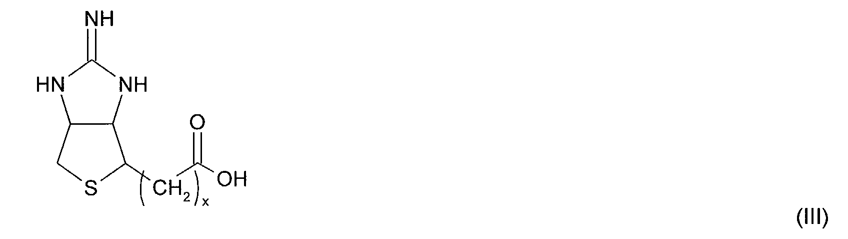 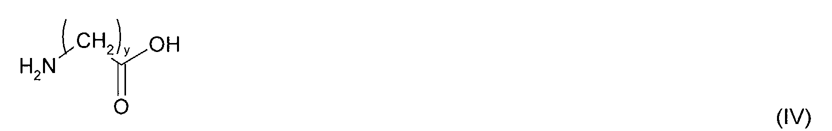 wobei x und y wie oben definiert sind.

6. Verfahren zur Herstellung eines Iminobiotinderivates nach einem der Ansprüche 2, 3 oder 4, mit der Stufe, in der man ein Iminobiotinderivat der allgemeinen Formel (I) mit 1-Ethyl-3-(3-dimethylaminopropyl)-carbodiimid (EDC) und Sulfo-N-hydroxysuccinimid (Sulfo-NHS) umsetzt.

7. Verfahren nach Anspruch 6, wobei die Umsetzung in phosphatgepufferter, wäßriger Lösung bei einem pH-Wert von 6,0 bis 8,5 für 30 Minuten bis 5 Stunden bei 0°C bis 50°C durchgeführt wird.

8. Verfahren zur Markierung von Makromolekülen mit primärer Aminofunktion mit einem Iminobiotinderivat, mit der Stufe, in der man ein Iminobiotinderivat der allgemeinen Formel (II) in wäßriger Lösung mit den Makromolekülen umsetzt, 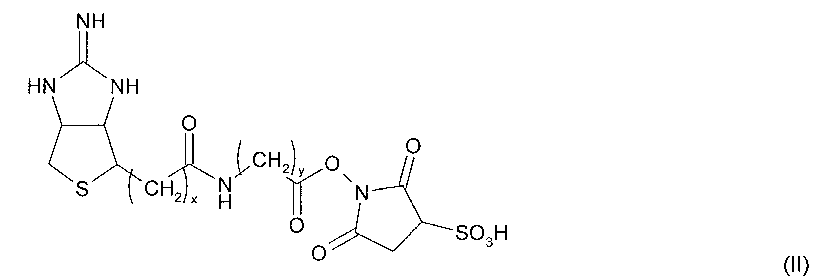 wobei x und y wie oben definiert sind.

9. Verfahren nach Anspruch 7, **dadurch gekennzeichnet, daß** man das Iminobiotinderivat der allgemeinen Formel (II) für die Markierung als Zwischenprodukt *in situ* aus dem Iminobiotinderivat der Formel (I) durch Umsetzung mit 1-Ethyl-3-(3-dimethylaminopropyl)-carbodiimid (EDC) und Sulfo-N-hydroxysuccinimid (Sulfo-NHS) herstellt.

10. Verwendung des Iminobiotinderivates nach einem der Ansprüche 1 bis 4 zur Markierung von Makromolekülen mit primärer Aminofunktion.

11. Verwendung nach Anspruch 10, **dadurch gekennzeichnet, daß** die Makromoleküle mit primärer Aminofunktion Proteine sind.

12. Verwendung nach Anspruch 11, **dadurch gekennzeichnet, daß** die Proteine Zellmembranproteine mit extrazellulär zugänglichen Proteinabschnitten sind.

13. Verfahren zur differentiellen qualitativen, semiquantitativen oder quantitativen Bestimmung von Proteinen oder anderen Makromolekülen mit primärer Aminofunktion in Zellen des gleichen Zelltyps, die sich in verschiedenen Zuständen befinden, oder in Zellen verschiedenen Zelltyps mit den Stufen, in denen man
a) Proteine oder Makromoleküle der Zellen mit dem Iminobiotinderivat der Formel (II) markiert, wobei man für Zellen in verschiedenen Zuständen bzw. für verschiedene Zelltypen jeweils verschiedene Isotopomere der Iminobiotinderivate mit verschiedenen Molekulargewichten einsetzt,
b) gegebenenfalls die markierten Proteine oder Makromoleküle aus Zellen verschiedener Zustände bzw. aus verschiedenen Zelltypen vereinigt,
c) die markierten Proteine oder Makromoleküle affinitätschromatographisch reinigt und/ oder anreichert und
d) die markierten Proteine oder Makromoleküle mittels massenspektrometrischer Methoden analysiert.

14. Verfahren nach Anspruch 13, **dadurch gekennzeichnet, daß** die verschiedenen Isotopomere der Iminobiotinderivate kein bzw. verschiedene Mengen an Deuterium (D) oder Tritium (T) im Austausch für Wasserstoff (H) enthalten.

15. Verfahren nach einem der Ansprüche 13 oder 14, **dadurch gekennzeichnet, daß** man in der Markierungsstufe a) intakte Zellen einsetzt und extrazellulär zugängliche Abschnitte von deren Membranproteinen markiert.
